# FASCICULE DE BREVET EUROPEEN

(11) **EP 0 807 449 B1**
(45) Date de publication et mention de la délivrance du brevet: **08.10.2003**
(21) Numéro de dépôt: 96203123.3
(22) Date de dépôt: 07.11.1996
(51) Int. Cl.: A61M 25/02

(54) **Pansement protecteur pour cathéters profonds et son nécessaire hygiénique**
Schutzpflaster für tiefliegende Katheter und Zubehör
Protection plaster for profound catheters and its necessities

(30) Priorité: 15.05.1996 FR 9606329
(43) Date de publication de la demande: 19.11.1997
(73) Titulaire: Vogel, Denise, 65000 Tarbes (FR)
(72) Inventeur: Vogel, Denise, 65000 Tarbes (FR)
(74) Mandataire: Ravina, Bernard

(56) Documents cités:
- EP-A- 0 286 525
- EP-A- 0 515 807
- EP-A- 0 671 182
- US-A- 5 372 589

## Description

La présente invention a trait au domaine médical et/ou sanitaire et concerne plus précisément un pansement protecteur pour les cathéters profonds, adapté aux traitements ambulatoires.

Le cathéter ou sonde désigne un tuyau mince, de préférence en plastique, souple et susceptible d'être introduit dans l'organisme (par exemple : une veine ou une artère) pour l'explorer ou pour y intervenir. L'introduction du cathéter dans un conduit appelé « cathétérisme » est une intervention délicate qui nécessite en outre de la part du personnel médical soignant, un respect de règles sanitaires et hygiéniques draconiennes afin de prévenir tout risque d'infection du patient.

Les cathéters visés par le pansement protecteur de l'invention sont ceux du type portés en permanence par des patients dialysés qui débouchent à l'extérieur de la peau et qui sont généralement au nombre de deux dont, un pour l'aspiration et l'autre pour le retour du liquide dans un circuit extérieur à l'organisme mis en place périodiquement pour séparer ledit liquide des substances toxiques avant de la réinjecter dans l'organisme.

Le port permanent de ce type de cathéter n'est pas sans poser de sérieux problèmes tant pour les dialysés que pour le personnel médical assurant les soins.

En effet, les embouts externes des cathéters peuvent constituer un foyer d'infection pour l'atmosphère, pour le personnel et pour les patients du lieu où sont prodigués les soins. Aussi, il est donc nécessaire, une fois l'intervention de dialyse achevée, de mettre en place un pansement sur les embouts saillants des cathéters de façon à minimiser le risque de contamination du patient, en limitant la durée de l'exposition à l'air libre desdits embouts. Le pansement de protection traditionnel utilisé à l'heure actuelle est encore rudimentaire et met en oeuvre des moyens de fortune souvent improvisés par le personnel soignant qui ne dispose pour l'instant d'aucun dispositif spécifique adapté à ce besoin. Ces moyens de fortune consistent à appliquer des compresses stériles sur les embouts de cathéters et à les maintenir en place plaquées sur la peau à l'aide de rubans adhésifs. Il s'ensuit de nombreux inconvénients et parmi ceux-ci :
- Une mise en place longue (de l'ordre de vingt minutes) et très lourde (enrobage des bouchons des embouts par des compresses bétadinées, pose de compresses stériles sur les embouts et application de rubans adhésifs occlusifs),
- Un manque de confort pour le patient ambulatoire équipé d'un tel pansement volumineux et inesthétique.
- Un risque de contamination de ce pansement non étanche à l'eau ou autre souillure du milieu ambiant environnant,
- Un temps important de préparation préalable pour la mise en place du circuit de dialyse puisque, pour dégager les embouts de cathéter pour la connexion, il est indispensable de supprimer le pansement avec toutes les incommodités que cela engendre pour le patient,
- Une augmentation du risque d'infection au moment de la connexion du circuit de dialyse sur les deux embouts des cathéters,
- L'impossibilité pour le patient de prendre des douches ou des bains sans risquer d'endommager son pansement,
- Etc.

Par ailleurs, il existe des dispositifs de protection de cathéter décrit notamment celui dans le document EP 0671182 (GAMBRO AB) constitué d'une poche munie d'une fenêtre circulaire avec des moyens d'adhésion disposés autour de la fenêtre pour rendre la poche hermétique, cette poche comportant en outre une ouverture refermable permettant le branchement et le débranchement des cathéters.

La demanderesse a mené des recherches ayant pour objet l'étude d'un pansement d'un type nouveau qui obvie aux inconvénients précités et qui assure la protection hermétique des embouts de cathéters profonds, dans des conditions d'hygiène et de confort satisfaisantes aussi bien pour le patient que pour le personnel soignant, tout en favorisant une large diffusion d'un tel pansement en veillant à son moindre prix de revient par une fabrication en série industrielle.

A cet effet le pansement selon l'invention, protecteur de cathéters profonds débouchant à l'extérieur de la peau d'un patient formé d'une poche (100) pouvant recevoir dans sa partie inférieure (120) les embouts (300a-300b) desdits cathéters, cette poche (100) comportant dans sa partie supérieure (110) sur la face (100a) destinée à venir au contact de la peau du patient, une fenêtre (110a) se caractérise essentiellement en ce que cette fenêtre (110a), est obturée par une bande constituée d'une compresse non tissée (210) munie de deux fentes (211a-212b) pour le passage des cathéters, ladite bande étant bordée d'un ruban (220) entourant au moins ladite fenêtre (110a) de la poche (100), la face (210a) de la dite bande susceptible d'être appliquée sur la peau étant aseptisée et la face (220a) dudit ruban (220) susceptible d'être appliquée sur la peau étant recouverte d'un matériau adhésif permettant de retenir la poche (100) sur la peau du patient.

Selon une autre caractéristique de l'invention, les dites faces de la dite bande et dudit ruban destinés à être appliqués sur la peau sont recouvertes par une pellicule protectrice siliconée détachable (200a) formée de deux languettes (201a-202a) terminées à leurs extrémités en vis-à-vis de deux rabats (201a-202b) se recouvrant pour être facilement saisissables.

La présence de cette poche, façonnée dans une matière thermoplastique souple transparente imperméable telle que le polyéthylène agréé médicalement, a pour objet, en position dite d'ouverture de sa fenêtre, d'autoriser le passage des embouts de cathéter à l'intérieur, et en position dite de fermeture, lorsque sa fenêtre est obturée par le moyen d'adhésion assujetti sur la peau autour desdits embouts, d'assurer le cloisonnement étanche de la poche contenant les embouts, préservant ainsi les principes de base de l'hygiène. Un tel pansement a pour avantage de concilier en un seul produit industriel, la protection étanche des embouts de cathéter et le moyen de retenue de la poche sur le corps du patient. Il en découle une réduction non négligeable des temps nécessaires à la mise ne place du pansement et à la connexion du circuit de dialyse puisqu'avec le pansement protecteur de l'invention, cette connexion sur les embouts de cathéter peut maintenant être exécutée selon un processus totalement différent, ne nécessitant plus la dépose du pansement. En effet, il suffit de pratiquer une découpe dans la poche sur le pansement en place, notamment dans sa partie inférieure opposée à celle où est pratiquée la fenêtre, pour accéder directement aux dits embouts et pour connecter à l'intérieur de la poche le circuit extérieur de traitement. En outre, ce pansement a des incidences appréciables pour le confort du patient tant sur le plan de l'aisance (la poche souple déformable n'est retenue à la peau que sur une surface réduite du moyen d'adhésion entourant la fenêtre), que sur le plan esthétique (discrétion du pansement sous les vêtements) ou encore que sur le plan sécurisant (peu de risque de contamination garantie par l'étanchéité du matériau polyéthylène de la poche).

L'invention se rapporte également à un nécessaire hygiénique de pansement protecteur pour deux cathéters profonds à des fins de dialyse, constitué d'un emballage renfermant :
- Un pansement protecteur selon l'invention,
- Un lot de compresses non tissées stériles,
- Un premier champ opératoire stérile avec fenêtre,
- Un deuxième champ opératoire stérile de table,
- Un emballage des compresses stériles,
- Et deux bouchons revêtus chacun de la couleur de l'embout du cathéter correspondant.

Ce nécessaire de pansement peu volumineux et prêt à être utilisé est indispensable et suffisant au personnel soignant pour exécuter dans de bonnes conditions d'hygiène le changement d'un pansement protecteur selon l'invention.

La description qui va suivre a pour but d'illustrer et d'imager les concepts fondamentaux de l'invention tels qu'évoqués ci-dessus dans leur forme la plus élémentaire et ce afin de laisser à l'homme de métier la possibilité d'en imaginer toutes les variantes adaptables à tout type de cathéter.

Cette description se réfère aux dessins annexés sur lesquels :

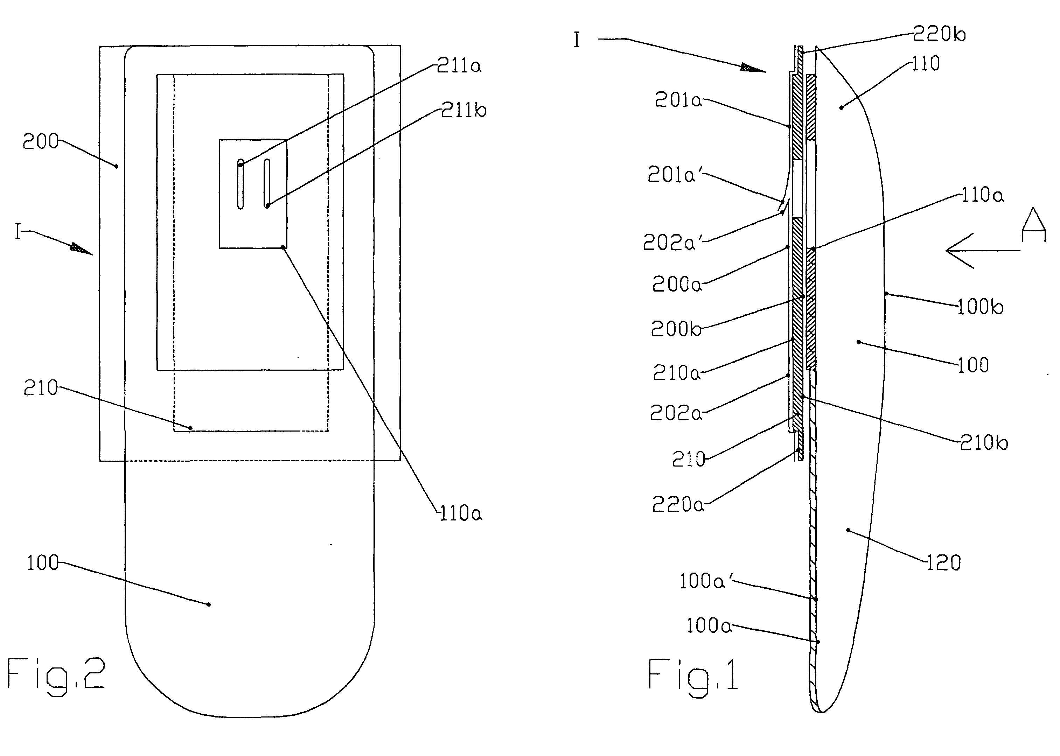

La figure 1 est une vue en coupe d'un mode de réalisation d'un pansement protecteur à usage unique pour deux embouts de cathéters conforme à l'invention.

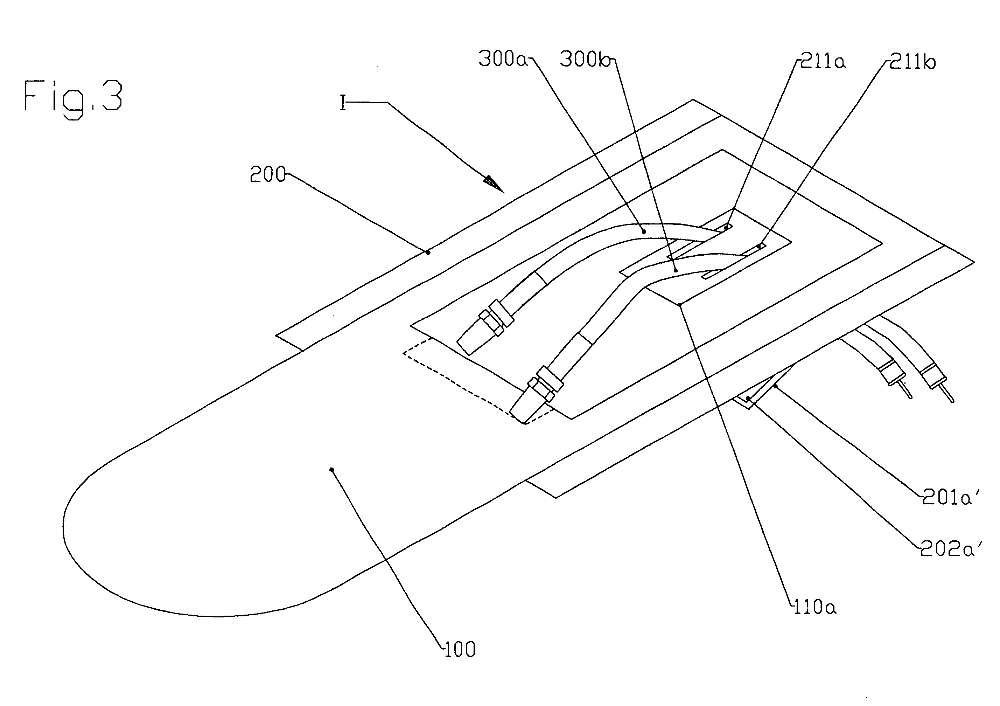

La figure 2 est une vue selon A du pansement de la figure 1.
La figure 3 est une vue en perspective de ce pansement avec les deux embouts de cathéter installés à l'intérieur.
La figure 4 est une vue d'un nécessaire hygiénique d'un tel pansement protecteur.
Tel qu'illustré sur l'ensemble des dessins, le pansement protecteur est constitué :
   - D'une poche 100 comportant une face 100a côté peau et d'une face 100b côté opposé,
   - D'un moyen d'adhésion 200 de la poche 100 à la peau du patient.

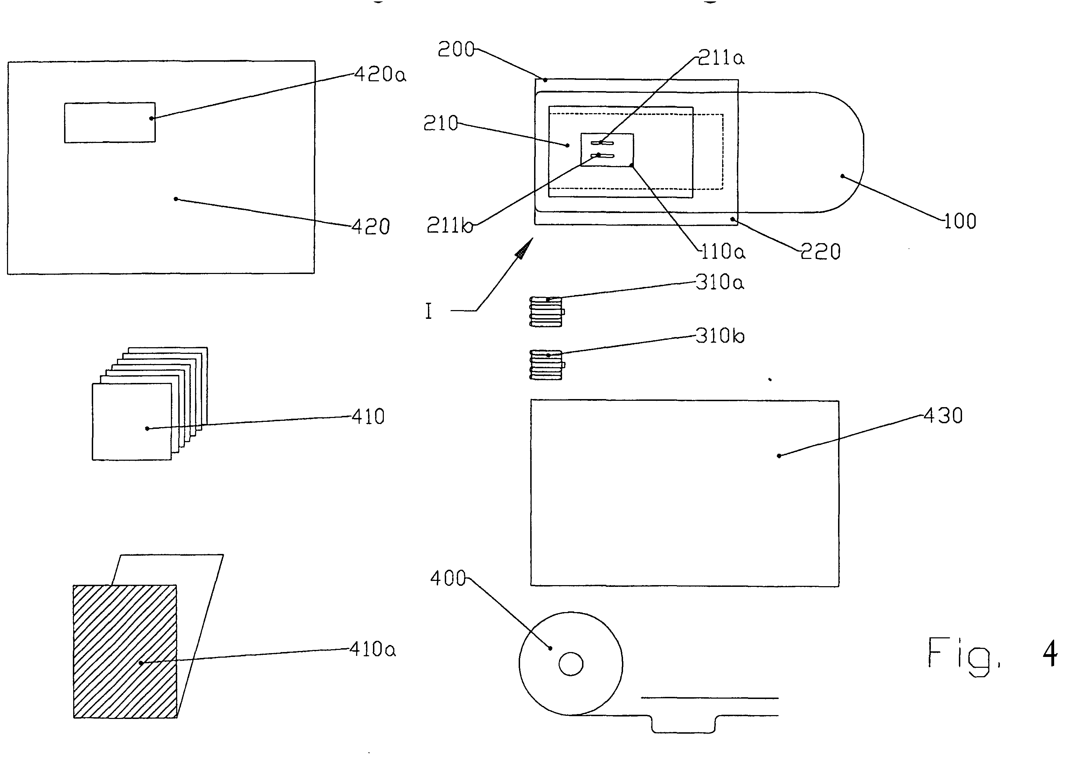

La poche 100, façonnée dans une matière thermoplastique souple transparente imperméable, tel que le polyéthylène agréé médicalement, est ouverte d'une fenêtre 110a (préférentiellement pratiquée dans la partie haute 110 de sa face 100a) pour laisser le passage de deux embouts de cathéter 300a et 300b débouchant à l'extérieur de la peau du patient et destiné, l'un (300a) à l'aspiration et l'autre (300b) au retour du liquide dans l'organisme, via un circuit extérieur de traitement connecté périodiquement sur lesdits embouts. Les extrémités de ces embouts 300a et 300b sont, soit connectées aux lignes d'un circuit de dialyse, soit fermées par deux bouchons amovibles 310a et 310b (cf. figure 4) revêtus chacun de la même couleur (bleu ou rouge) que celle de l'embout correspondant 300a et 300b.

Le susdit moyen d'adhésion 200 est disposé au moins autour de la fenêtre 110a, afin de rendre hermétique la poche 100 appliquée sur la peau avec ses embouts de cathéter 300a et 300b emprisonnés à l'intérieur. La souplesse de la matière de la poche 100 lui permet de suivre les mouvements des embouts autour de sa fixation sur la peau, laquelle est réduite à la surface adhésive dudit moyen 200. Le fait d'ouvrir la fenêtre 110a dans la partie supérieure 110 de la poche 100 destinée à être en contact avec la peau permet de laisser libre la partie inférieure 120 de la poche avec ses embouts de cathéter 300a et 300b qui est naturellement retenue suspendue à la peau par son moyen d'adhésion 200. En outre, en pratiquant une découpe à la base de la partie inférieure 120 de la poche 100 en place, le personnel soignant pourra accéder directement aux embouts 300a et 300b et immédiatement sans dépose du pansement pour assurer, après désinfection, la connexion du circuit de dialyse à l'intérieur de la poche 100 formant encore enveloppe de protection pendant l'intervention.

La face 100a de la poche 100 pourra être recouverte d'un voile 100a' en matériau non adhérent agréable au toucher, disposé autour dudit moyen d'adhésion 200. L'autre face 100b de la poche 100 restera transparente pour visualiser rapidement des problèmes de saignement, de suintement, de coloration de la peau, etc...

Le susdit moyen d'adhésion 200 se compose, quant à lui, d'une bande centrale 210 formée d'une compresse non tissée et percée d'une ouverture formée dans le cas présent de deux fentes 211a et 211b parallèles à l'axe longitudinal de la poche 100 pour laisser le passage des deux embouts de cathéter 300a et 300b. Cette compresse 210 formant la bande centrale est bordée d'un ruban périphérique 220 entourant au moins la fenêtre 110 de la poche 100. La face 210a de la compresse de la bande centrale susceptible d'être appliquée sur la peau est aseptisée et la face 220a du ruban 220 susceptible d'être appliquée sur la peau est recouverte d'un matériau adhésif permettant de retenir la poche 100 sur la peau autour desdits embouts 300a et 300b.

Selon une caractéristique particulièrement avantageuse de l'invention, la face 210a de la compresse de la bande centrale 210 et celle 220a du ruban adhésif 220, destinées à être en contact avec la peau, sont recouvertes d'une pellicule protectrice détachable 200a, façonnée classiquement dans du papier siliconé. Cette pellicule 200a se compose de deux languettes 201a et 202a, disposées perpendiculairement à l'axe longitudinal de la poche 100, et terminées à leurs extrémités en vis-à-vis de deux petits rabats 201a' et 202a' se recouvrant pour être facilement saisissables et pouvoir retirer facilement les deux languettes 201a et 202a, l'une après l'autre après avoir engagé les deux embouts 300a et 300b dans la poche 100, via les deux fentes 211a et 211b et la fenêtre 110a.

Selon une autre caractéristique particulièrement avantageuse, la face 210b de la compresse de la bande centrale 210 et celle 220b du ruban adhésif 220 en vis-à-vis de la poche 100, sont recouvertes d'un voile anti-adhérent imperméable de protection 200b.

Le dessin de la figure 4 montre un nécessaire hygiénique de pansement protecteur pour deux cathéters profonds d'aspiration dont doit être équipé au minimum le personnel soignant pour respecter les consignes d'hygiène d'installation d'un circuit de dialyse. Tel qu'illustré ce nécessaire comprend un emballage 400 destiné à renfermer :
- Une poche 100 avec son moyen d'adhésion 200 comportant une bande centrale 210 percée de deux fentes parallèles 211a et 211b parallèles à l'axe longitudinal de la poche 100 et un ruban adhésif 220 entourant au moins la fenêtre 110a de la poche 100,
- Un lot de compresses non tissées stériles 410,
- Un premier champ opératoire stérile 420 avec fenêtre 420a,
- Un deuxième champ opératoire stérile de table 430,
- Un emballage 410a des compresses 410,
- Deux bouchons 310a et 310b revêtus chacun de la couleur de l'embout de cathéter correspondant 300a et 300b.

## Revendications

1. Pansement protecteur de cathéters profonds débouchant à l'extérieur de la peau d'un patient formé d'une poche (100) pouvant recevoir dans sa partie inférieure (120) les embouts (300a-300b) desdits cathéters, cette poche (100) comportant dans sa partie supérieure (110) sur la face (100a) destinée à venir au contact de la peau du patient, une fenêtre (110a) **caractérisé en ce que** cette fenêtre (110a), est obturée par une bande constituée d'une compresse non tissée (210) munie de deux fentes (211a-211b) pour le passage des cathéters, ladite bande étant bordée d'un ruban (220) entourant au moins ladite fenêtre (110a) de la poche (100), la face (210a) de la dite bande susceptible d'être appliquée sur la peau étant aseptisée et la face (220a) dudit ruban (220) susceptible d'être appliquée sur la peau étant recouverte d'un matériau adhésif permettant de retenir la poche (100) sur la peau du patient.

2. Pansement protecteur selon la revendication 1 **caractérisé en ce que** les deux fentes (211a-211b) prévues dans la compresse (210) obturant la fenêtre (110a) sont orientées parallèlement à l'axe longitudinal de la poche (100).

3. Pansement protecteur selon la revendication 1 **caractérisé en ce que** lesdites faces de ladite bande et dudit ruban destinées à être appliquées sur la peau sont recouvertes par une pellicule protectrice siliconée, détachable (200a) formée de deux languettes (201a-202a) terminées à leurs extrémités en vis-à-vis de deux rabats (201a-202b) se recouvrant pour être facilement saisissables.

4. Nécessaire hygiénique de pansement renfermant :
- Un pansement protecteur selon les revendications 1 à 3 (100).
- Un lot de compresses non tissées stériles (410).
- Un premier champ opératoire stérile (420) avec fenêtre (420a).
- Un deuxième champ opératoire stérile de table (430).
- Un emballage (410a) des compresses stériles (410).
- Deux bouchons (310a et 310b) revêtus chacun de la couleur de l'embout de cathéter correspondant (300a et 300b).

## Patentansprüche

1. Schutzpflaster für tiefliegende Katheter, die außerhalb der Haut eines Patienten münden, gebildet von einer Tasche (100), welche in ihrem unteren Teil (120) die Anschlüsse (300a-300b) der Katheter aufnehmen kann, wobei diese Tasche (100) in ihrem oberen Teil (110) an der zum Kontakt mit der Haut des Patienten bestimmten Seite (100a) ein Fenster (110a) aufweist, **dadurch gekennzeichnet, daß** dieses Fenster (110a) verschlossen ist durch einen Streifen, der aus einer ungewebten Kompresse (210) besteht, die mit zwei Schlitzen (211a-211b) für den Durchtritt der Katheter versehen ist, wobei der Streifen von einem Band (220) umgeben ist, das mindestens das Fenster (110a) der Tasche (100) umgibt, wobei die zum Anbringen auf der Haut vorgesehene Seite (210a) des Streifens keimfrei gemacht ist und die zum Anbringen auf der Haut vorgesehene Seite (220a) des Bandes (220) mit einem Klebstoff versehen ist, welcher das festhalten der Tasche (110) auf der Haut des Patienten ermöglicht.

2. Schutzpflaster nach Anspruch 1, **dadurch gekennzeichnet, daß** die zwei Schlitze (211a-211b), die in der das Fenster (110a) umgebenden Kompresse (210) vorgesehen sind, parallel zur Längsachse der Tasche (100) ausgerichtet sind.

3. Schutzpflaster nach Anspruch 1, **dadurch gekennzeichnet, daß** die zum Anbringen auf der Haut vorgesehenen Seiten des Streifens und des Bandes mit einer abnehmbaren silikonisierten Schutzhaut (200a) abgedeckt sind, die von zwei Zungen (201 a-202a) gebildet ist, die an ihren gegenüberliegenden Enden in zwei Fortsätzen (201 a-202b) enden, die sich überdecken, damit sie leicht ergreifbar sind.

4. Hygienisches Schutzpflasterset umfassend:
- Ein Schutzpflaster nach den Ansprüchen 1 bis 3 (100).
- Eine Anzahl ungewebter steriler Kompressen (410).
- Ein erstes steriles Operationsfeld (420) mit Fenster (420a).
- Eine zweites steriles Tischoperationsfeld (430).
- Ein Verpackung (410a) der Kompressen (410).
- Zwei Stopfen (310a und 310b), die jeder mit der Farbe des entsprechenden Katheteranschlusses (300a und 300b) überzogen sind.

## Claims

1. A protective dressing for deep catheters opening out outside the skin of a patient, formed by a pouch (100) able to receive the end pieces (300a-300b) of such catheters in its bottom part (120), said pouch (100) comprising in its top part, (110) on the face (100a), intended to come into contact with the skin of the patient, a window (110a), **characterised in that** said window (110a) is closed off by a strip formed of a non-woven compress (210) provided with two slits (211a-211b) for the passage of catheters, said strip being bordered by a ribbon (220) surrounding at least said window (110a) in the pouch (100), the face (210a) of said strip suitable to be applied to the skin being asepticised and the face (220a) of said ribbon (220) suitable to be applied to the skin being covered with an adhesive material for holding the pouch (100) on the skin of the patient.

2. A protective dressing according to claim 1, **characterised in that** the two slits (211a-211b) provided in the compress (210) closing off the window (110a) are oriented parallel to the longitudinal axis of the pouch (100).

3. A protective dressing according to claim 1, **characterised in that** said faces of said strip and of said ribbon intended to be applied to the skin are covered with a detachable silicone-coated protective film (200a) formed by two tongues (201a-202a) terminating at their opposite ends in two flaps (201a-202b) overlapping so as to be easily grippable.

4. A Hygienic dressing case containing:
- a protective dressing according to claims 1 to 3 (100)
- a batch of sterile non-woven compresses (410)
- a first sterile operative area (420) with window (420a)
- a second sterile table operative area (430)
- a package (410a) for the sterile compresses (410)
- two plugs (310a and 310b) each covered with the colour of the corresponding catheter end piece (300a and 300b).
